# EUROPÄISCHE PATENTANMELDUNG

(11) **EP 4 151 139 A1**
(43) Veröffentlichungstag der Anmeldung: **22.03.2023**
(21) Anmeldenummer: 22192679.3
(22) Anmeldetag: 29.08.2022
(51) Int. Cl.: A61B 1/04, A61B 1/00

(54) **VERFAHREN UND VORRICHTUNG ZUR VIDEOENDOSKOPIE MIT FLUORESZENZLICHT**

(30) Priorität: 16.09.2021 DE 102021124010
(71) Anmelder: XION GmbH, 13127 Berlin (DE)
(72) Erfinder: Müller, Holger, 13127 Berlin (DE)
(74) Vertreter: Eisenführ Speiser

(57) **Zusammenfassung**

Die Erfindung betrifft ein Verfahren zum Durchführen einer Videoendoskopie mit Fluoreszenzlicht. Das Verfahren umfasst die folgenden Schritte:
- Aufnehmen einer ersten Bildfolge, die sich aus zeitlich aufeinanderfolgen Einzelbildern zusammensetzt, mit einem endoskopischen Videosystem,
- Aufnehmen einer zweiten Bildfolge, die sich aus zeitlich aufeinanderfolgen Fluoreszenzbildern zusammensetzt, mit demselben endoskopischen Videosystem,
- Bilden einer Transformationsfunktion zwischen verschiedenen Einzelbildern der ersten Bildfolge,
- Anwenden der Transformationsfunktion auf den Einzelbildern der ersten Bildfolge zugeordnete, aufeinanderfolgende Fluoreszenzbilder der zweiten Bildfolge zum Gewinnen transformierter Fluoreszenzbilder,
- Überlagern eines aktuellen Fluoreszenzbilds der zweiten Bildfolge mit wenigstens einem oder mehreren transformierten Fluoreszenzbildern, die aus dem aktuellen Fluoreszenzbild in der zweiten Bildfolge unmittelbar vorangehenden Fluoreszenzbildern gewonnen wurden, um ein verbessertes Fluoreszenzbild zu gewinnen, und
- Darstellen eines jeweiligen, derart verbesserten Fluoreszenzbilds, so dass sich eine verbesserte zweite Bildfolge ergibt, die sich aus verbesserten Fluoreszenzbildern zusammensetzt.

## Beschreibung

### Gegenstand der Erfindung

Die Erfindung betrifft ein Verfahren und eine Vorrichtung zur Videoendoskopie mit Fluoreszenzlicht. Unter einer Vorrichtung zur Videoendoskopie wird auch ein Kamerakopf mit auswechselbar angeschlossen Endoskopen verstanden und somit nicht nur - aber auch - ein Videoendoskop im engeren Sinne. Unter einem Videoendoskop im engeren Sinne wird meist ein Endoskop mit fest eingebauter Kamera verstanden.

### Bekannter Stand der Technik

Videosysteme zur endoskopischen Darstellung von Fluoreszenzbildern sind bekannt. Häufig werden sie bei chirurgischen Operationen verwendet. Beispielsweise kann von Krebs befallenes Gewebe durch einen fluoreszierenden Farbstoff eingefärbt werden. Durch das Fluoreszenzleuchten ist das kranke Gewebe für den operierenden Arzt besser von gesundem Gewebe unterscheidbar und kann sicherer operativ entfernt werden.

Endoskopische Videosysteme zur Aufnahme von Fluoreszenzbildern können typischerweise auch Bilder unter sichtbarem Licht aufnehmen. Dazu wird ein Objekt mit sichtbarem Licht beleuchtet und das von dem Objekt reflektierte sichtbare Licht aufgenommen. Eine Bildfolge derart aufgenommener Einzelbilder ergibt dann eine Videosequenz, die die Erscheinung des Objekts unter sichtbarem Licht wiedergibt. Gleichzeitig oder abwechselnd kann ein Objekt auch mit fluoreszenz-anregender Strahlung bestrahlt werden, um eine zweite Bildfolge mit Fluoreszenzbildern aufzunehmen. Einzelbilder, die unter sichtbaren Licht aufgenommen wurden und die erste Bildfolge bilden, werden im Folgenden schlicht als Einzelbilder (im engeren Sinne) oder als Reflexionsbilder bezeichnet, während Einzelbilder der zweiten Bildfolge, die unter fluoreszenz-anregender Strahlung aufgenommen wurden, als Fluoreszenzbilder bezeichnet werden.

Die Einzelbilder und Fluoreszenzbilder der beiden Bildfolgen können gleichzeitig aufgenommen werden, wenn sich die Wellenlängen der Fluoreszenz und/oder der fluoreszenzanregenden Strahlung von der des für die Reflexionsbilder verwendeten sichtbaren Lichts unterscheiden. Mittels optischer Filter können so ein Fluoreszenzkanal und ein Reflexionskanal (d.h. der Kanal für reflektiertes Licht) voneinander getrennt werden, auch wenn beide Kanäle aus jeweils gelichzeitig aufgenommen Einzel- bzw. Fluoreszenzbilder der zugehörigen ersten und zweiten Bildfolge gebildet sind.

Es ist auch möglich, immer abwechselnd ein Einzelbild oder mehrere Einzelbilder unter sichtbarem Licht und ein Fluoreszenzbild oder mehrere Fluoreszenzbilder aufzunehmen, wobei der Zeitversatz zwischen dem Einzelbild und dem zugehörigen Fluoreszenzbild nur sehr gering ist. Der Fluoreszenzkanal und der Reflexionskanal sind somit zeitlich voneinander getrennt, weil die Einzel- bzw. Fluoreszenzbilder der zugehörigen ersten und zweiten Bildfolge zeitlich zueinander versetzt sind.

In beiden Fällen wird üblicherweise zusätzlich ein optisches Sperrfilter vor der Kamera eingesetzt, um das vom Objekt reflektierte Anregungslicht zu sperren, da dieses reflektierte Anregungslicht sonst das durch Fluoreszenz emittierte Licht überstrahlen würde.

Die Helligkeit eines Fluoreszenzbildes ist bei der Endoskopie üblicherweise sehr gering. Deswegen wird auch bei Nutzung hochempfindlichen Bildsensoren eine starke Signalverstärkung benötigt, um das Fluoreszenzleuchten wiederzugeben. In US 2004/0210107 A1 und US 8,721,532 B2 wird deswegen die Addition benachbarter Pixel des Fluoreszenzbildes zur Signalverstärkung vorgeschlagen. Durch die Addition steigt zwar die Helligkeit von großen fluoreszierenden Flächen. Gleichzeitig sinkt dadurch die optische Auflösung des Fluoreszenzbildes. Folglich sind mit dieser Methode feine Objektstrukturen nur schlecht erfassbar.

In US 8,428,318 B2 werden in einer Videosequenz von Fluoreszenzbildern auf die Bilder ein Rauschfilter und ein Convolution-Filter angewendet. Dann werden die Bilder durch eine affine Transformation bewegungskorrigiert und zur weiteren Rauschunterdrückung eine Mittelwertbildung der Bilder vorgenommen. Diese Methode eignet sich wegen der Tiefpasswirkung der Filter ebenfalls nur für die Verbesserung der Wiedergabe von groben Strukturen die großflächig fluoreszieren. Ferner ist infolge des starken Bildrauschens des Fluoreszenzbildes die Berechnung der affinen Transformation nur beim Vorhandensein stark fluoreszierender Strukturen im Bild möglich.

In US 4,821,117 A wird die Verstärkung des Fluoreszenzbildes durch einen Bildverstärker vorgeschlagen. Nachteil ist hierbei der durch den Bildverstärker bedingte hohe technische Aufwand. Bildverstärker besitzen außerdem meist nur eine geringe Auflösung. Deswegen verbessert auch diese Anordnung nicht die Wiedergabe feiner, schwach fluoreszierender Strukturen.

Der Erfindung liegt die Aufgabe zugrunde, ein Verfahren und eine Vorrichtung zu schaffen, die eine verbesserte Videoendoskopie mit Fluoreszenzlicht erlaubt.

Erfindungsgemäß wird hierfür ein Verfahren mit den folgenden Schritten vorgeschlagen:
- Aufnehmen einer ersten Bildfolge (Videosequenz), die sich aus zeitlich aufeinanderfolgen Einzelbildern zusammensetzt, mit einem endoskopischen Videosystem, indem ein Objekt beleuchtet und das von dem Objekt reflektierte Licht als Einzelbild aufgenommen wird
- Aufnehmen einer zweiten Bildfolge (Videosequenz), die sich aus zeitlich aufeinanderfolgen Fluoreszenzbildern zusammensetzt, mit demselben endoskopischen Videosystem, indem das Objekt mit fluoreszenz-anregender Strahlung bestrahlt und das von dem Objekt ausgehende Licht als Fluoreszenzbild aufgenommen wird
   - wobei das Aufnehmen der ersten und der zweiten Bildfolge - und somit der Einzelbilder und der Fluoreszenzbilder - entweder gleichzeitig erfolgt, oder abwechselnd ein Einzelbild oder mehrere Einzelbilder und ein Fluoreszenzbild oder mehrere Fluoreszenzbilder aufgenommen werden, so dass jedem Einzelbild ein unmittelbar zuvor oder unmittelbar danach - und damit wenigstens annähernd zeitgleich - aufgenommenes Fluoreszenzbild zugeordnet werden kann,
- Identifizieren einer Transformationsfunktion - im einfachsten Fall einer linearen Verschiebung - zwischen aufeinanderfolgenden Einzelbildern,
- Anwenden der Transformationsfunktion auf den Einzelbildern zugeordnete, aufeinanderfolgende Fluoreszenzbilder zum Gewinnen transformierter Fluoreszenzbilder,
- Überlagern eines aktuellen Fluoreszenzbilds der zweiten Bildfolge mit wenigstens einem oder mehreren transformierten Fluoreszenzbildern, die aus dem aktuellen Fluoreszenzbild in der zweiten Bildfolge vorangehenden Fluoreszenzbildern gewonnen wurden um ein verbessertes Fluoreszenzbild zu gewinnen, und
- Darstellen eines jeweiligen, derart verbesserten Fluoreszenzbilds, so dass sich eine verbesserte zweite Bildfolge ergibt, die sich aus verbesserten Fluoreszenzbildern zusammensetzt.

Das Verfahren läuft somit so ab, dass zunächst mit einem Videoendoskop zwei Videosequenzen gleichzeitig oder quasi gleichzeitig aufgenommen werden. Eine erste Videosequenz wird aus einer ersten Bildfolge gebildet, die sich aus Einzelbildern zusammensetzt, die von einem Objekt unter Verwendung des von diesem Objekt reflektierten Lichts gemacht werden. Parallel dazu - entweder genau gleichzeitig oder abwechselnd mit den Einzelbildern der ersten Bildfolge - werden von dem Objekt Bilder bei Beleuchtung des Objekts mit Fluoreszenz-anregender Strahlung aufgenommen, um so eine zweite Videosequenz zu erstellen, die von einer zweiten Bildfolge gebildet wird, die sich aus Fluoreszenzbildern zusammensetzt. Als Fluoreszenzbilder werden hier die Einzelbilder bezeichnet, die von einem Objekt unter Verwendung des vom Objekt durch Fluoreszenz emittierten Lichts gemacht werden.

Dadurch dass die Einzelbilder der ersten Bildfolge und damit der ersten Videosequenz gleichzeitig oder abwechselnd mit dem Fluoreszenzbildern der zweiten Bildfolge und damit der zweiten Videosequenz aufgenommen werden, kann jedem Fluoreszenzbild entweder ein gleichzeitig aufgenommenes Einzelbild der ersten Bildfolge zugeordnet werden oder - falls die Einzelbilder und Fluoreszenzbilder abwechselnd aufgenommen werden - jedem Fluoreszenzbild wenigstens ein unmittelbar vorangehendes oder nachfolgendes Einzelbild zugeordnet werden. Da das Verhältnis der Anzahl von Einzelbildern und Fluoreszenzbildern nicht zwangsläufig eins zu eins sein muss, sondern beispielsweise in einem sich wiederholenden Zyklus auch jeweils zwei Reflexionsbilder und ein Fluoreszenzbild - oder umgekehrt - aufgenommen werden können. Kann ein Fluoreszenzbild auch zwei Einzelbildern zugeordnet sein - oder umgekehrt, ein Einzelbild zwei Fluoreszenzbildern.

Da die Einzelbilder und die Fluoreszenzbilder mit dem gleichen Videoendoskop gleichzeitig oder quasi gleichzeitig aufgenommen werden, zeigen einander zugeordnete Einzel- und Fluoreszenzbilder auch jeweils die gleiche oder annähernd gleiche Szene aus gleicher oder annähernd gleicher Perspektive. Das gilt auch dann, wenn beispielsweise das Endoskop bewegt wird oder sich das Objekt bewegt. In beiden Fällen unterscheiden sich aufeinander folgende Einzelbilder der ersten Bildfolge aufgrund der Bewegung des Endoskops oder des Objekts. In gleicher Weise oder annähernd gleicher Weise unterscheiden sich auch aufeinanderfolgende, den Einzelbildern zuzuordnende Fluoreszenzbilder der zweiten Bildfolge. Die Unterschiede zwischen zwei aufeinander folgenden Einzelbildern können allein darin bestehen, dass die Abbildungen des Objekts in den beiden Einzelbildern relativ voneinander verschoben und/oder verdreht sind. Aufgrund von Verzeichnungen der Endoskopoptik - beispielsweise tonnenförmiger Verzeichnung - oder einer Bewegung des Objekts, die sich nur in einem Teil des aufgenommenen Bildes wiederspielt, ist es aber auch möglich, dass sich zwei aufeinanderfolgende Einzelbilder nicht einfach durch lineare Verschiebungen oder Verdrehungen voneinander unterscheiden, sondern dass die Unterschiede zwischen zwei aufeinanderfolgenden Einzelbildern durch eine komplexere Transformationsfunktion beschrieben werden müssen, die die für verschiedene Bereiche des Einzelbildes unterschiedlichen Verschiebungen, Verdrehungen, Streckungen oder Stauchungen abbilden. In jedem dieser Fälle ist es mit an sich bekannten Mitteln möglich, die Transformationsfunktion zu identifizieren, mit der ein Einzelbild der ersten Bildfolge in ein vorangehendes oder nachfolgendes Einzelbild der ersten Bildfolge überführt werden kann.

Der Erfindung liegt die Idee zugrunde, dass die zum Identifizieren der Transformationsfunktion herangezogenen Bildmerkmale in den Einzelbildern der ersten Bildfolge besser und genauer identifiziert werden können, da die Einzelbilder der ersten Bildfolge selbst bei geringer Tiefenschärfe üblicherweise genügend klar zu identifizierende Bild- oder Objektmerkmale zeigen. Somit können aus den Einzelbildern der ersten Bildfolge Transformationsfunktionen bestimmt werden, die beschreiben, wie - d.h. mit welcher Transformationsfunktion - ein Einzelbild der ersten Bildfolge auf ein anderes Einzelbild derselben Bildfolge abgebildet werden kann, sodass Bild- und Objektmerkmale sich am gleichen Ort befinden.

Die gleichen Transformationsfunktionen können auf aufeinander folgende Fluoreszenzbilder der zweiten Bildfolge angewandt werden. Dies ist besonders einfach, wenn Reflexionsbilder und Fluoreszenzbilder jeweils zeitgleich aufgenommen wurden. Wenn Reflexionsbilder und Fluoreszenzbilder zeitsequentiell - also z.B. abwechselnd oder zyklisch - aufgenommen werden, kann eine zeitliche Interpolation nötig sein, um eine für den Aufnahmezeitpunkt des Fluoreszenzbildes passende Transformation zu errechnen. Mit dem erfindungsgemäßen Verfahren wird in jedem Fall die Notwendigkeit einer Detektion von Objekten im Fluoreszenzbild vermieden. Dementsprechend läuft das Verfahren so ab, dass die Transformationsfunktionen zwischen aufeinander folgenden Einzelbildern auf Basis der Einzelbilder der ersten Bildfolge gebildet werden und diese Transformationsfunktionen dann auf die entsprechenden Fluoreszenzbilder der zweiten Bildfolge angewandt werden. Dabei kann beispielsweise auch die direkte Transformation zum jeweils letzten Bild und damit auch die entsprechende Transformationsfunktion gebildet werden. Das Anwenden der Transformationsfunktion kann eine Interpolation beinhalten, falls Einzelbilder und Fluoreszenzbilder abwechselnd oderzyklisch - und somit zeitversetzt- aufgenommen werden, Auf diese Weise werden ein oder mehrere positionsverbesserte, transformierte Fluoreszenzbilder gewonnen, die sich dann überlagern lassen, sodass sich dann hinsichtlich des Signal-Rauschverhältnisses verbessertes Fluoreszenzbild ergibt.

Entsprechend kann jedes Fluoreszenzbild der zweiten Bildfolge mit ein, zwei oder mehreren transformierten, vorangehenden Fluoreszenzbildern überlagert und so verbessert werden, sodass sich im Ergebnis eine verbesserte zweite Bildfolge ergibt, die sich aus verbesserten Fluoreszenzbildern zusammensetzt. Auf diese Weise wird mit dem Verfahren eine verbesserte Videosequenz mit Fluoreszenzbildern gewonnen.

Gemäß einem weiteren Aspekt wird eine Vorrichtung vorgeschlagen, die ein Videoendoskopiesystem mit einer Bildverarbeitungseinrichtung umfasst. Das Videoendoskopiesystem ist dazu konfiguriert, zwei Bildfolgen aufzunehmen, und zwar eine erste Bildfolge, die sich aus zeitlich aufeinanderfolgen Einzelbildern zusammensetzt, wobei ein Objekt beleuchtet und das von dem Objekt reflektierte Licht als Einzelbild aufgenommen wird, und eine zweite Bildfolge, die sich aus zeitlich aufeinanderfolgen Fluoreszenzbildern zusammensetzt, wobei das Objekt mit fluoreszenz-anregender Strahlung bestrahlt und das von dem Objekt ausgehende Licht als Fluoreszenzbild aufgenommen wird. Die Bildverarbeitungseinrichtung ist dazu konfiguriert,
- ein oder mehrere Einzelbilder der ersten Bildfolge einem oder mehreren, wenigstens annähernd gleichzeitig aufgenommen Fluoreszenzbildern der zweiten Bildfolge zuzuordnen,
- aus den Einzelbildern der ersten Bildfolge Transformationsfunktionen abzuleiten, die die geometrische Veränderung von Bildmerkmale zwischen zwei Einzelbildern beschreiben,
- die abgeleiteten Transformationsfunktionen auf die den Einzelbildern entsprechenden Fluoreszenzbilder anzuwenden und
- die auf diese Weise transformierten Fluoreszenzbilder zu einem verbesserten Fluoreszenzbild zu überlagern.

Das Videoendoskopiesystem ist gemäß einer Variante dazu konfiguriert, zwei Bildfolgen derart aufzunehmen, dass das Aufnehmen der Einzelbilder und der Fluoreszenzbilder der ersten beziehungsweise der zweiten Bildfolge gleichzeitig erfolgt, so dass jedem Einzelbild ein gleichzeitig aufgenommenes Fluoreszenzbild zugeordnet werden kann.

Das Videoendoskopiesystem ist gemäß einer zweiten Variante dazu konfiguriert, zwei Bildfolgen derart aufzunehmen, dass abwechselnd ein Einzelbild oder mehrere Einzelbilder und ein Fluoreszenzbild oder mehrere Fluoreszenzbilder aufgenommen werden, so dass jedem Einzelbild ein Fluoreszenzbild zugeordnet werden kann, welches mit geringem zeitlichen Versatz zum Einzelbild vor oder nach dem Einzelbild aufgenommen wurde

Die Erfindung berücksichtigt, dass bei der Videoendoskopie das auf den Bildsensor fallende Fluoreszenzlicht generell sehr schwach ist. Schwach fluoreszierende Objekte lassen sich deswegen besonders schlecht wiedergeben. Die schwache Fluoreszenz liegt nahe an der Nachweisschwelle und ist kaum vom Bildrauschen des Lichtempfängers zu unterscheiden. Die insbesondere in der Astronomie übliche Verlängerung der Belichtungszeit ist in der chirurgischen Endoskopie nicht praktikabel. Denn ein endoskopisches Videosystem soll Bewegungen von Objekten im Bild mit hoher Bildrate flüssig wiedergeben. Nur so ist anhand des Echtzeitbildes auf dem Monitor eine optimale Handhabung des Endoskops und der chirurgischen Instrumente möglich.

Bekannte Methoden und Geräte können zwar die Wiedergabe von schwach leuchtender Fluoreszenz verbessern, bei der Verbesserung der Lichtempfindlichkeit wird aber ungewollt die optische Bildauflösung verschlechtert. Infolgedessen ist die Erkennbarkeit schwach fluoreszierender Strukturen auf größere Strukturen mit ausreichender Ausdehnung der Fluoreszenz beschränkt. Kleine Strukturen werden nur wiedergegeben, wenn sie hell fluoreszieren. Diagnostisch interessante feine Strukturen weisen aber vielfach schon aufgrund ihrer geringen Ausdehnung nur schwache Fluoreszenz auf.

Infolgedessen lassen sich solche feinen, schwach fluoreszierenden Strukturen bei Nutzung der bekannten Methoden und endoskopischen Videosysteme nicht wiedergeben.

Endoskope werden in der Praxis oft zur Bilddarstellung mit sehr kleinen Objektweiten verwendet. Dadurch wird ein ausreichender Abbildungsmaßstab zur Erkennung kleiner Objekte erreicht. Die Tiefenausdehnung der im Bild befindlichen Objekte kann dabei deutlich die Schärfetiefe überschreiten und bereits geringe Bewegungen des Endoskops können zu großen perspektivischen Veränderungen des endoskopischen Bildes führen. Daneben können biologische Objekte wie das Herz sich nicht nur bewegen, sondern auch dynamisch Ihre Form und ihr Aussehen ändern.

Bekannte Methoden der Bildverbesserung, Bildstabilisierung und Videoverbesserung funktionieren unter diesen Bedingungen nur unzureichend.

Ziel der Erfindung ist es, diese vom bekannten Stand der Technik nicht gelösten Probleme zu überwinden und die Wiedergabe feiner, schwach fluoreszierender Objektstrukturen durch Videoendoskopie zu verbessern.

Erfindungsgemäß wird die Aufgabe dadurch gelöst, dass simultan oder zeitsequentiell in einem Bildkanal für reflektiertes Licht und einem Bildkanal für Fluoreszenzlicht Bilder als Video erfasst werden und auf Grundlage der Analyse der Einzelbilder des Bildkanals für reflektiertes Licht eine Verbesserung eines jeweiligen Fluoreszenzbildes vorgenommen wird.

So kann beispielsweise das im Folgenden in seinen Schritten beschriebene Verfahren zur Anwendung gebracht wird.

In der ersten Bildfolge des Bildkanals des Reflexionslichtes werden in mindesten zwei verschiedenen Einzelbildern der ersten Bildfolge Bild- und/oder Objektmerkmale identifiziert, welche auch im zuletzt erfassten Einzelbild der mindestens zwei Einzelbilder vorkommen.

Die Merkmalsposition der erkannten Bild- und/oder Objektmerkmale wird in einer Genauigkeit erfasst, die der Größe der mit dem endoskopischen Videosystem darstellbaren Feinstruktur entspricht oder noch genauer ist.

Zum Erkennen von Bildmerkmalen und Erfassen ihrer Merkmalsposition können alle bekannten Verfahren der Merkmalserkennung oder Objekterkennung genutzt werden. Beispielsweise eignen sich Algorithmen wie SIFT (Scale Invariant Feature Transform), SURF (Speeded Up Robust Features) oder Blockbildung aus Teilflächen des Bildes zum Identifizieren von Bildmerkmalen.

Die Positionsveränderungen dieser identifizierten Merkmalspositionen werden relativ zum zeitlich letzten Einzelbild der mit reflektiertem Licht aufgenommenen ersten Bildfolge berechnet. Der Begriff Positionsveränderung wird hier im weiteren Sinne verwendet und kann neben der Veränderung der Pixelposition auch Merkmale wie Drehwinkel, Abbildungsmaßstab oder Verzerrung einschließen. Es wird aus den ermittelten Positionsveränderungen von dem oder den zeitlich früheren Einzelbildern zum zeitlich letzten Einzelbild für jedes frühere Einzelbild jeweils eine geometrische Bildtransformation gebildet, welche den ermittelten Positionsveränderungen entspricht. Aus einer so gebildeten geometrischen Bildtransformation wird die zugehörige Transformationsfunktion abgeleitet.

Die diese geometrische Transformation oder geometrischen Transformationen von dem oder den vorangehenden, unter reflektiertem Licht aufgenommenen Einzelbild zum zeitlich letzten unter reflektiertem Licht aufgenommenen Einzelbild beschreibende Transformationsfunktion wird auf das oder die jeweils zeitgleich oder zeitnah belichteten Fluoreszenzbilder der zweiten Bildfolge, also Einzelbilder der Bildfolge des Bildkanals für Fluoreszenz, angewendet. Durch das Anwenden der aus der unter reflektiertem Licht aufgenommen Bildfolge gewonnenen geometrischen Transformation auf die entsprechenden Fluoreszenzbilder des Fluoreszenzkanals können diese Fluoreszenzbilder mit zeitlich vorangehenden Fluoreszenzbildern (also Einzelbildern des Fluoreszenzkanals) passgenau überlagert werden um zu verbesserten Fluoreszenzbildern des Fluoreszenzkanals zu gelangen.

Durch Anwenden jeder Transformationsfunktion entsteht jeweils ein Fluoreszenzbild, in welchem feine Objektstrukturen mit dem zeitlich letzten Fluoreszenzbild besser als beim geometrisch nicht transformierten Fluoreszenzbild übereinstimmen.

Dieses verbesserte Fluoreszenzbild oder diese verbesserten Fluoreszenzbilder der zweiten Bildfolge werden gemeinsam zur Berechnung eines verbesserten Fluoreszenzbilds der zweiten Bildfolge verwendet. Diese Verbesserung kann beispielsweise durch Addition oder Mittelwertbildung der Grauwerte der positionsverbesserten Fluoreszenzbilder und des zeitlich letzten Fluoreszenzbildes erfolgen. Weiterhin kann neben räumlicher Filterung auch zeitliche Filterung der positionsverbesserten Fluoreszenzbilder erfolgen.

Durch Wiederholen dieses in seinen Schritten beschriebenen Ablaufs entsteht aus den verbesserten Fluoreszenzbildern eine verbesserte zweite Bildfolge und somit ein verbessertes Fluoreszenzvideo.

Das Erkennen der Bild- und/oder Objektmerkmale in den Reflexionsbildern (also den Einzelbildern der ersten Bildfolge) kann dadurch vereinfacht werden, dass die Positionsveränderung zum letzten Einzelbild der ersten Bildfolge indirekt aus den Positionsveränderungen zwischen zeitlich direkt aufeinanderfolgenden Einzelbildern der ersten Bildfolge ermittelt wird. Durch den geringen zeitlichen Versatz unterscheiden sich diese Einzelbilder nur minimal, was das Finden übereinstimmender Merkmal wesentlich vereinfacht. Die geometrische Transformation zum letzten Einzelbild der ersten Bildfolge kann dann gebildet werden, indem die geometrischen Transformationen zwischen den zeitlich aufeinander folgenden Einzelbildern der ersten Bildfolge hintereinander ausgeführt werden bis das zeitlich letzte Einzelbild der ersten Bildfolge erreicht ist. Entsprechend können auch die die Transformationen beschreibenden Transformationsfunktionen hintereinander ausgeführt und auf die aufeinanderfolgenden Fluoreszenzbilder angewandt werden.

Alternativ kann das Verfahren in Stufen angewendet werden. Dazu wird aus zwei zeitlich aufeinanderfolgenden Einzelbildern der ersten Bildfolge ein positionskorrigiert gemitteltes Einzelbild gebildet. Dieses gemittelte Einzelbild wird geometrisch transformiert und mit dem zeitlich letzten Einzelbild der ersten Bildfolge und gegebenenfalls weiteren gemittelten Einzelbildern positionskorrigiert gemittelt. Dabei kann eine Wichtung gemäß der Anzahl der in einer Mittelungsstufe gemittelten Anzahl an Einzelbildern erfolgen.

Die genannte geometrische Transformationsfunktion kann im einfachsten Fall eine Bildverschiebung beschreiben. Für diese einfachste mögliche Bildverbesserung reicht das Erfassen der Positionsveränderung einer einzelnen Merkmalposition. Durch eine affine Transformation ist eine bessere Kompensation von Relativbewegungen zwischen Endoskop und Objekt möglich. Es können jedoch auch nichtlineare geometrische Transformationen verwendet werden. Dann kann mit dem erfindungsgemäßen Verfahren auch an sehr beweglichen Objekten wie dem Herzmuskel oder den Stimmlippen die Sichtbarkeit von feinen Strukturen im Fluoreszenzbild verbessert werden.

Zur Erhöhung der Robustheit des erfindungsgemäßen Verfahrens können mehrere Merkmalspositionen durch Mittelwertbildung oder andere statistische Methoden, wie beispielsweise RANSAC (random sample consensus), zusammengefasst werden. Hierbei ist auch das Entfernen von durch fehlerhafte Erfassung von Merkmalspositionen verursachten Ausreißern möglich.

In einer besonders vorteilhaften Auslegung werden bei der Bestimmung der geometrischen Transformationsfunktion Merkmalspunkte von stark strukturierten Objekten und/oder Merkmalspunkte nahe der Bildmitte stärker gewichtet oder ausschließlich verwendet. Stark strukturierte Objekte können beispielsweise durch eine höhere Dichte von erfassbaren Merkmalspunkten identifiziert werden.

In einer besonders vorteilhaften Auslegung wird vor der Auswertung der Merkmalspositionen eine Kompensation der optischen Verzeichnung des Endoskops des endoskopischen Videosystems vorgenommen. Das kann durch Entzerren des jeweiligen Einzelbildes unter Anwendung der inversen Verzeichnung auf das Einzelbild vor dem Erkennen der Bild- und/oder Objektmerkmale oder direkt durch Umrechnen der Merkmalsposition der erfassten Bild- und/oder Objektmerkmale erfolgen. Durch die Entzerrung wird die statistische Auswertung der Positionsveränderungen deutlich verbessert. Das gilt insbesondere dann, wenn lineare geometrische Transformationen zur Bildverbesserung eingesetzt werden. Das Ergebnis der Verbesserung des Fluoreszenzbildes kann in diesem Fall dadurch erzielt werden, dass bei der Anwendung der geometrischen Transformation zur Berechnung des geometrisch verbesserten Fluoreszenzbildes das Bild wieder eine der optischen Verzeichnung des Endoskops entsprechende Verzerrung des Fluoreszenzbildes erfolgt. Alternativ können auch die Bildkanäle von Fluoreszenz und Reflexion nach der Bilderfassung und vor dem Detektieren von Bildmerkmalen und dem Identifizieren der Transformationsfunktion entzerrt werden und die oben genannte Methode auf den entzerrten Bildern zur Anwendung gelangen. Dabei ist dann auch die entzerrte Darstellung beider Bildkanäle einfach möglich.

Gemäß einer Ausführungsvariante werden die Einzelbilder (Reflexionsbilder) und die Fluoreszenzbilder der ersten bzw. zweiten Bildfolge zeitsequentiell erfasst. Zur Kompensation des zeitlichen Versatzes zwischen Reflexionsbild und zugehörigem Fluoreszenzbild innerhalb der beiden Videobildfolgen wird eine zeitliche Interpolation und/oder zeitlich Extrapolation der ermittelten Positionsveränderungen der identifizierten Bildmerkmale durchgeführt und die geometrische Transformation oder geometrischen Transformationen jeweils aus diesen interpolierten und/oder extrapolierten Positionsveränderungen gebildet oder es wird eine zeitliche Interpolation und/oder Extrapolation der geometrischen Transformationen gebildet. Besonders Vorteilhaft ist die Anwendung dieser Ausführungsvariante bei einer Bilderfassung durch einen Farbsensor, der zeitsequentiell das Reflexionsbild und das Fluoreszenzbild erfasst.

Besonders vorteilhaft ist die Implementierung des Verfahrens in einem Echtzeit-Bildgebungssystem für chirurgische Operationen, wo der Operateur zur motorischen Steuerung feiner Handbewegungen eine schnelle Bildgebung benötigt, was eine hohe Bildrate von beispielsweise 60 Bildern pro Sekunde erfordert. Mit solch hohen Bildraten geht eine geringe Belichtungszeit für jedes Fluoreszenzbild einher. Durch die geringe Belichtungszeit ist das Bildrauschen besonders störend. Die Implementierung kann beispielsweise auf der Basis von GPU, ASIC oder FPGA erfolgen.

Gemäß einer weiteren Ausführungsvariante werden in den Einzelbildern der ersten Bildfolge viele möglichst weit über das Bild verteilte Merkmalspositionen erfasst und die geometrische Transformationsfunktion dadurch gebildet, dass an Positionen zwischen den erfassten Merkmalspositionen die geometrische Transformation durch Interpolation der Positionsveränderungen an umliegenden Merkmalspositionen berechnet wird. An den Bildrändern kann diese nichtlineare geometrische Transformation durch Extrapolation der benachbarten Merkmalspositionen erfolgen und die Transformationsfunktion entsprechend gebildet werden.

Gemäß einem weiteren Aspekt wird das erfindungsgemäße Verfahren auf die Stereoendoskopie angewendet. Dazu können die Merkmalspositionen in beiden stereoskopischen Halbbildern in der oben für nicht stereoskopische Bilder beschriebenen Weise verbessert werden.

In einer Ausführungsvariante werden in zwei zeitgleich oder zeitnah aufgenommenen stereoskopischen Halbbildern des Bildkanals für Reflexion Merkmalspositionen identifiziert, die zu demselben Objektmerkmal gehören und daraus die stereoskopische geometrische Transformation vom linken zum rechten stereoskopischen Einzelbild berechnet. Die geometrische Transformation wird in diesem Fall aus den Positionsabweichungen berechnet, welche sich aus dem perspektivischen Unterschied zwischen dem rechten und linken Teil-Bildkanal ergeben. Bei einer ausreichend hohen Dichte solcher Merkmalspositionen kann eine Addition oder Mittelwertbildung zwischen dem linken und dem rechten Fluoreszenzbild im Bildkanal der Fluoreszenz gebildet werden. Durch eine stereoskopische Addition ist eine Verdoppelung der Empfindlichkeit möglich. Nach der Addition kann durch eine inverse geometrische Transformation auch das zweite verbesserte stereoskopische Halbbild gebildet werden.

Besonders vorteilhaft ist es hierbei, die Linsenverzeichnung durch Entzerren zu kompensieren, den Abbildungsmaßstab zwischen linkem und rechtem Bild anzugleichen und beim Auftreten einer durch mechanische Toleranzen verursachten vertikalen Disparität der stereoskopischen Bilder, diese vertikale Disparität auszugleichen. Zur Berechnung dieser Korrekturen können die identifizierten Merkmalspunkte genutzt werden.

Dadurch können mit dem erfindungsgemäßen Verfahren gemäß dem weiteren Aspekt stereoskopische Reflexionsbilder und Fluoreszenzbilder in einer mit hoher Genauigkeit justierten stereoskopisch Zentralprojektion auf dem Wiedergabegerät dargestellt werden. Hiermit wird eine sehr gute stereoskopische Wiedergabe erreicht und es werden die für schlecht justierte Stereoskopie charakteristischen Ermüdungserscheinungen bei der Dauerbetrachtung des Stereobildes vermieden.

Das für nicht stereoskopische Bildgebung beschriebene erfindungsgemäße Verfahren gemäß dem ersten Aspekt kann mit diesem erfindungsgemäßen stereoskopischen Verfahren gemäß dem weiteren Aspekt kombiniert werden, um eine weitere Verbesserung der Sichtbarkeit von kleinen Strukturen im Fluoreszenzbild zu erreichen.

Die Erfindung soll nun anhand eines Ausführungsbeispiels mit Bezug auf die Figuren näher erläutert werden. Diese zeigen in
- Figur 1:: ein Videoendoskopiesystem für die Fluoreszenzvideoendoskopie, mit welchem parallel Videosequenzen unter sichtbarem Licht und Videosequenzen mit Fluoreszenzbildern aufgenommen werden können;
- Figur 2a und b:: schematisiert dargestellte Einzelbilder einer ersten Bildfolge, die unter sichtbarem Licht aufgenommen wurde und Fluoreszenzbilder einer zweiten Bildfolge, die unter fluoreszenz-anregender Strahlung aufgenommen wurde, zur Illustration des erfindungsgemäßen Verfahrens;
- Figur 3a und b:: schematische Darstellungen der Bildveränderungen zwischen aufeinanderfolgenden Einzelbildern der ersten und zweiten Bildfolge analog zu Figuren 2a und 2b und der zugehörigen Transformationsfunktionen (Verschiebungen);
- Figur 4a und b:: die Anwendung der Transformationsfunktionen auf die Einzelbilder der zweiten Bildfolge (Figur 4a) das Überlagern der transformierten Einzelbilder der zweiten Bildfolge zu einem verbesserten Fluoreszenzbild;
- Figur 5:: eine schematische Darstellung eines einzelnen Fluoreszenzbildes mit einer kleinen, schwach im Fluoreszenzlicht leuchtenden Struktur;
- Figur 6:: eine schematische Darstellung eines Einzelbildes der ersten Bildfolge mit einem Merkmal im reflektierten Licht;
- Figur 7:: eine schematische Darstellung einer Überlagerung der reflektierenden Struktur aus Figur 5 und fluoreszierenden Strukturen;
- Figur 8:: eine schematische Darstellung des einzelnen Fluoreszenzbildes aus Figur 5, wie es auf einem Bildsensor erscheint;
- Figur 9:: eine schematische Darstellung einer Addition zweier überlagerter Fluoreszenzbilder bei idealer Bildgebung ohne Bildrauschen;
- Figur 10:: eine schematische Darstellung einer Addition zweier überlagerter Fluoreszenzbilder, wie sie von einem Sensor mit Bildrauschen erfasst werden.
- Figur 11:: eine schematische Darstellung einer mit dem erfindungsgemäßen Verfahren durchgeführten, positionsrichtigen Addition zweier überlagerter Fluoreszenzbilder;
- Figur 12:: eine schematische Darstellung einer Überlagerung eines Einzelbildes der ersten Bildfolge, das reflektierende Strukturen zeigt, mit einem einzelnen Fluoreszenzbild, das fluoreszierende Strukturen zeigt, unter Einfluss des Bildrauschens des Sensors;
- Figur 13:: eine erfindungsgemäße Bildüberlagerung der reflektierenden und fluoreszierenden Strukturen, die das Bildrauschen vermindert und die fluoreszierende Struktur erkennbar macht; und
- Figur 14:: eine schematische Darstellung des Ablaufs eines erfindungsgemäßen Verfahrens.

Ein endoskopisches Videosystem 10 - hier auch Videoendoskopiesystem - weist typischerweise ein Endoskop 12 und einen Kamerakopf 14 auf. Das Endoskop 12 ist über eine lösbare Kopplung 16 mit dem Kamerakopf 14 verbunden.

Der Kamerakopf 14 weist ein Objektiv 18 auf, das dazu dient, von dem Endoskop 12 gelieferte Bilder auf einem Bildsensor abzubilden.

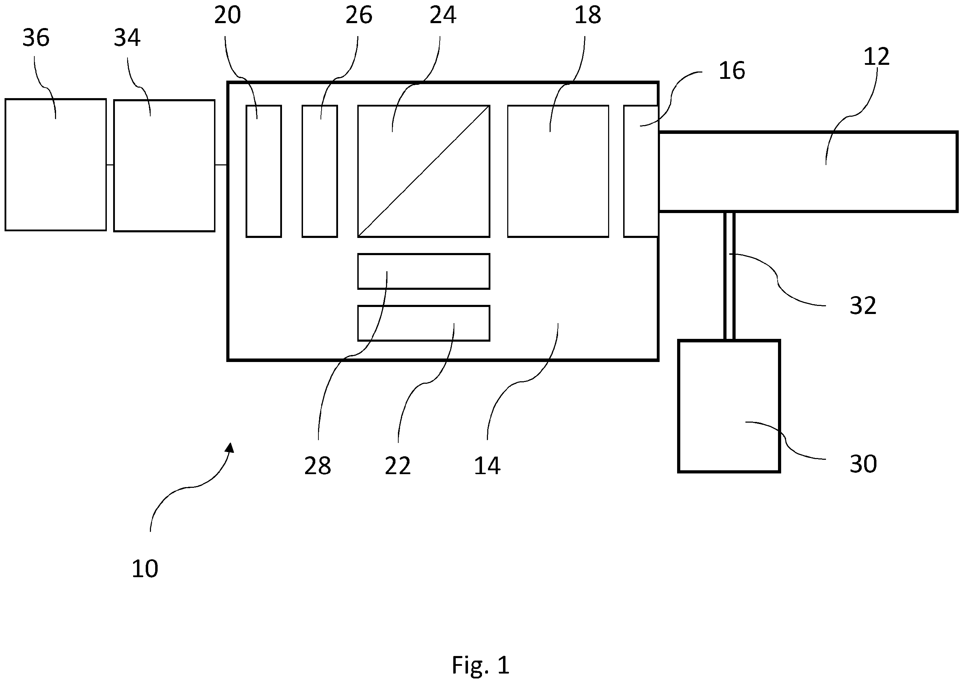

In dem in Figur 1 abgebildeten Fall weist der Kamerakopf 14 zwei Bildsensoren auf, nämlich einen Bildsensor 20 für sichtbares Licht und einen Bildsensor 22 für infrarote Fluoreszenzbilder. Um sowohl Einzelbilder bei sichtbarem Licht mit dem Bildsensor 20 als auch Fluoreszenzbilder mit dem Bildsensor 22 aufnehmen zu können, ist ein Strahlteiler 24 vorgesehen, der dazu führt, dass das Objektiv 18 die optischen Bilder sowohl auf dem Bildsensor 20 für sichtbares Licht als auch auf dem Bildsensor 22 für Fluoreszenzbilder abbildet. Um die auf diese Weise gebildeten zwei Bildkanäle optisch voneinander zu trennen, ist vor dem Bildsensor 20 für sichtbares Licht ein Sperrfilter 26 für infrarotes Licht vorgesehen, also ein Sperrfilter, der infrarotes Licht sperrt und für sichtbares Licht transparent ist. Entsprechend ist vor dem Bildsensor 22 für die Fluoreszenzbilder ein Sperrfilter 28 vorgesehen, der sichtbares Licht und die Fluoreszenz anregendes Licht sperrt.

Um ein mit dem Endoskop 12 zu betrachtendes Objekt - das sich beispielsweise in einer Körperhöhle befinden kann - zu beleuchten, ist eine Lichtquelle 30 vorgesehen die über einen Lichtleiter 32 Licht in das Endoskop 12 einspeist, sodass dieses Licht am distalen Ende des Endoskops 12 austreten kann. Die Lichtquelle 30 stellt sowohl sichtbares Licht bereit, als auch Licht mit einer Wellenlänge, die für die Anregung der Fluoreszenz geeignet ist.

Während des Betriebs werden mit den beiden Bildsensoren 20 und 22 vorzugsweise gleichzeitig zwei Bildfolgen aufgenommen. Mit dem Bildsensor 20 für sichtbares Licht wird eine erste Bildfolge aufgenommen, die sich aus Einzelbilder zusammensetzt und mit dem Bildsensor 22 für Fluoreszenzbilder wird eine zweite Bildfolge aufgenommen, die sich aus Fluoreszenzbildern zusammensetzt. In diesem Fall ist jedem mit dem Bildsensor 20 aufgenommenen Einzelbild auch ein gleichzeitig mit dem Bildsensor 22 aufgenommenes Fluoreszenzbild zugeordnet.

Die die Bildfolgen repräsentierenden Signale werden von dem Kamerakopf einer Bildverarbeitungseinrichtung 34 zugeführt, die die Einzel- und Fluoreszenzbilder wie nachfolgend beschrieben verarbeitet. Die von der Bildverarbeitungseinrichtung 34 erzeugten, verbesserten Bilder der Bildfolgen können schließlich auf einem Bildschirm 36 angezeigt werden, und zwar auf Grund der nachfolgend beschriebenen Bildverarbeitung so, dass Einzelbilder und Fluoreszenzbilder so überlagert werden können, dass die Fluoreszenz auf dem Bildschirm 36 erkennbar ist. Dazu kann das Fluoreszenzleuchten beispielsweise elektronisch eingefärbt zu dem im reflektierten Licht aufgenommenen Farbbild addiert werden.

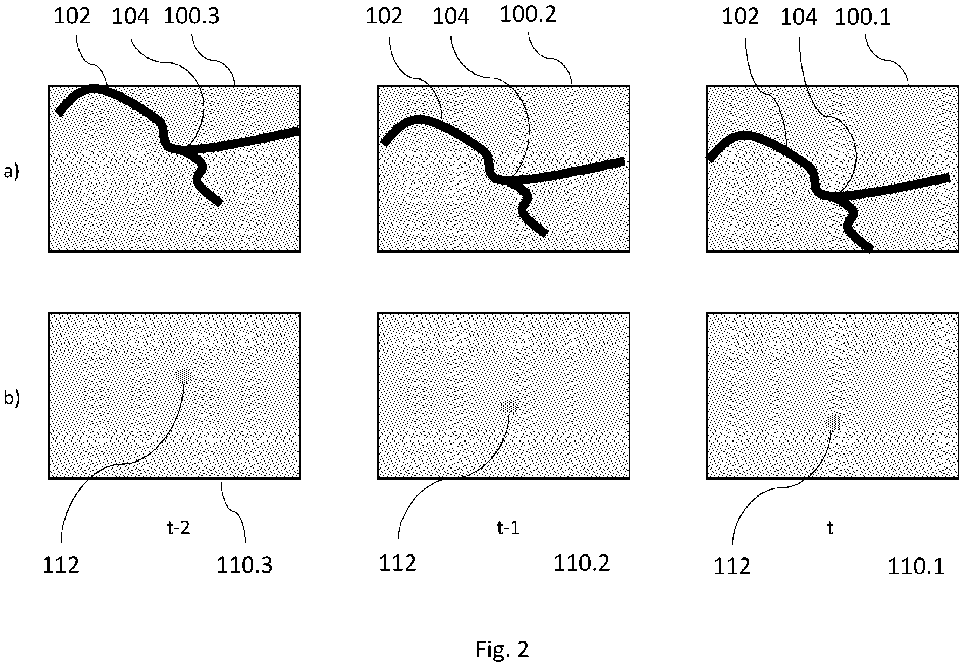

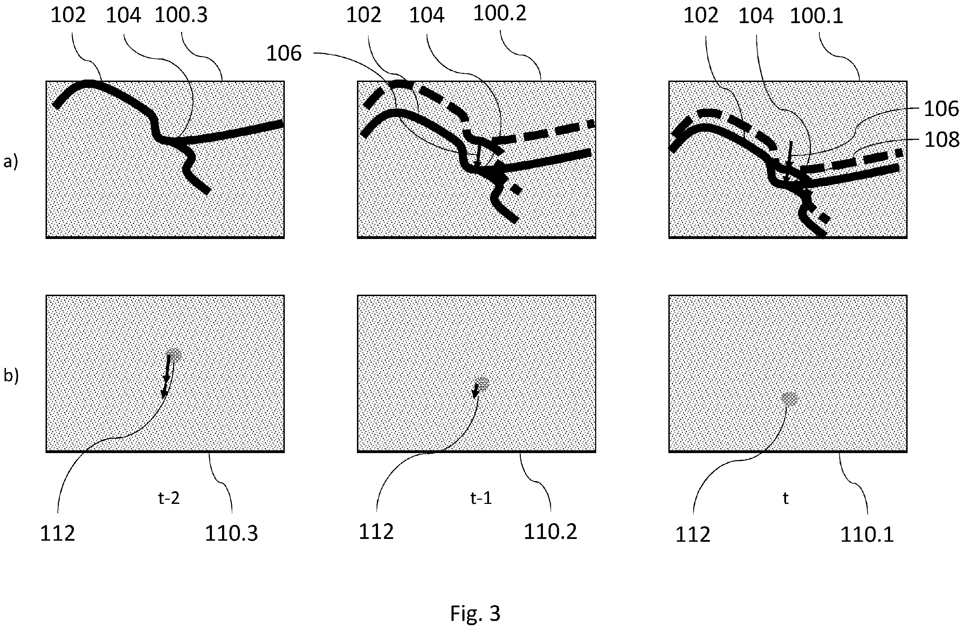

In Figur 2a sind abstrakt und schematisch drei aufeinanderfolgende Einzelbilder 100 einer ersten Bildfolge dargestellt, die bei Beleuchtung eines Objekts mit sichtbaren Licht aufgenommen wurden. Dargestellt ist ein aktuelles Einzelbild 100.1 zum Zeitpunkt t sowie ein vorangehendes Einzelbild 100.2 zum Zeitpunkt t-1 sowie noch ein vorangehendes Einzelbild 100.3 zum Zeitpunkt t-2. Wie Figur 3a zu entnehmen ist, unterscheiden sich die Einzelbilder 100.1, 100.2 und 100.3 zu den verschiedenen Zeitpunkten dadurch, dass das in den Einzelbildern 100 abgebildete Objekt 102 verschoben ist. Anhand leicht zu identifizierender Objektmerkmale (z.B. der Strukturverzweigung 104) kann die Verschiebung 106 bzw. 108 bestimmt werden, durch die sich zwei aufeinander folgende Einzelbilder 100 voneinander unterscheiden. Die so identifizierte Verschiebung 106 bzw. 108 ergibt eine Transformationsfunktion, mit der ein Einzelbild 100 zu einem Zeitpunkt passgenau auf ein Einzelbild 100 zu einem anderen (früheren oder späteren) Zeitpunkt abgebildet werden kann.

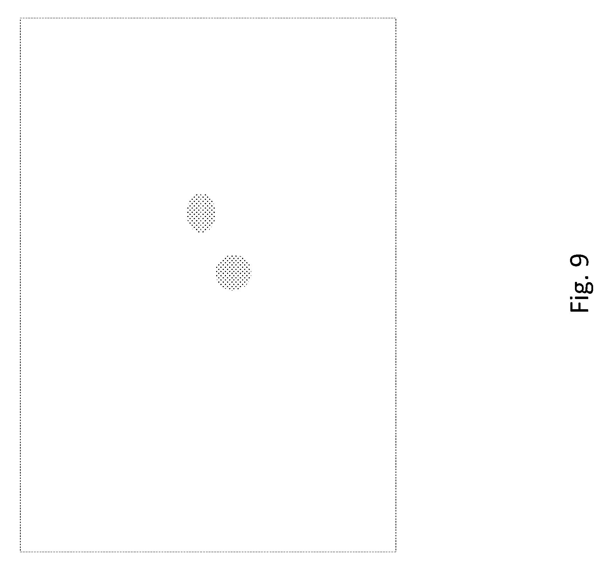

Figur 2b zeigt drei Fluoreszenzbilder 110 in der zweiten Bildfolge, die bei Bestrahlung mit Fluoreszenz-anregender Strahlung aufgenommen wurde. Die Fluoreszenz 112 tritt dabei nur lokal auf und ist in einem Einzelbild nur schwach zu erkennen. Ein verbessertes - insbesondere hinsichtlich des Signal-Rauschverhältnisses verbessertes - Fluoreszenzbild ließe sich durch Überlagern mehrerer Fluoreszenzbilder 110 (was in etwa auch einer längeren Belichtungszeit gleichkommt) erzeugen. Problematisch ist jedoch, dass sich das fluoreszierende Objekt 112 in den aufeinander folgenden Fluoreszenzbildern einer zweiten Bildfolge nicht immer am gleichen Bildort befindet; siehe Figur 9.

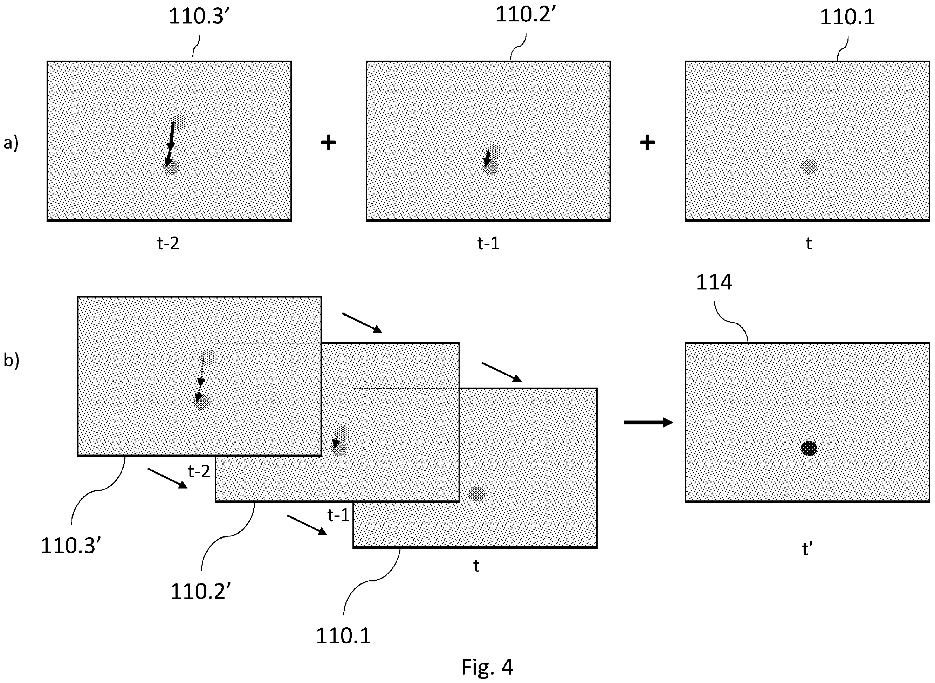

Daher ist es vorgesehen, aufeinander folgende Fluoreszenzbilder 110 der zweiten Bildfolge zunächst zu transformieren, um sie dann anschließend überlagern zu können. Dies ist in Figuren 3a, 3b, 4a und 4b schematisch dargestellt. Die zum Transformieren der Fluoreszenzbilder erforderlichen Transformationsfunktionen werden dabei aus den aufeinander folgenden Einzelbildern 100 der ersten Bildfolge gewonnen, und zwar beispielsweise mit an sich bekannten Verfahren wie SIFT oder SURF. Figur 3a zeigt, wie sich in jedem Einzelbild der ersten Bildfolge ein charakteristisches Objektmerkmal 104 identifizieren lässt. Dadurch kann für jeden Übergang von einem Einzelbild 100 zum nächsten Einzelbild ein 100 Verschiebungsvektor 106 bzw. 108 für eine lineare Verschiebung als einer einfachen Transformationsfunktion bestimmt werden. Die Verschiebungsvektoren, die die Transformationsfunktion definieren, sind in Figur 3a als Pfeile eingezeichnet.

Figur 3b zeigt, dass die gleichen Verschiebungen 106 und 108 auch für die entsprechenden Fluoreszenzbilder 110 gelten.

Daher können einem aktuellen Fluoreszenzbild 110.1 zum Zeitpunkt t vorangehende Fluoreszenzbilder 110.2 u d 110.3 zu den Zeitpunkten t-1 und t-2 so transformiert werden, dass deren Bildmerkmale im Ergebnis positionsverbessert und wenigstens annähernd deckungsgleich sind, siehe Figur 4a. Dann können die transformierten Fluoreszenzbilder 110.2' und 110.3' mit dem jeweils aktuellen Fluoreszenzbild 110.1 überlagert werden, um ein verbessertes aktuelles Fluoreszenzbild 114 mit verbessertem Signal-Rausch-Verhältnis zu erlangen; siehe Figur 4b.

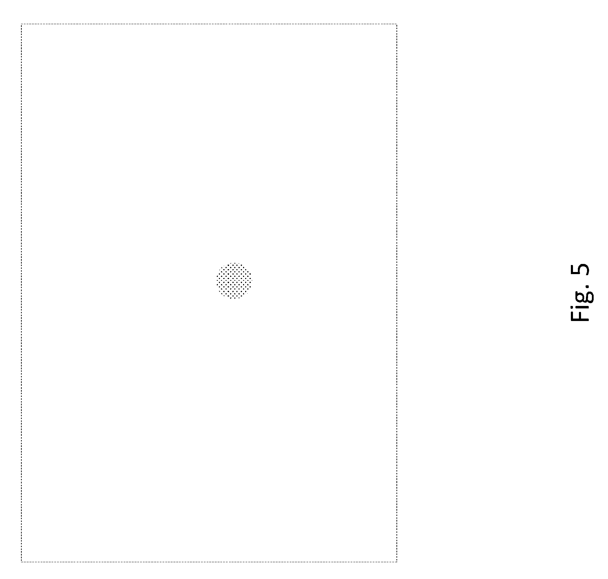

Figur 5 zeigt eine schematische Darstellung eines einzelnen Fluoreszenzbildes 110 mit einer kleinen, schwach im Fluoreszenzlicht leuchtenden Struktur 112 bei idealer Bildgebung ohne Bildrauschen.

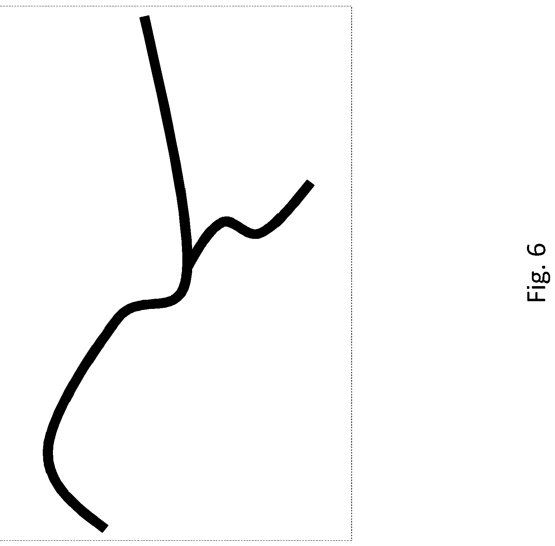

Figur 6 zeigt eine schematische Darstellung eines Einzelbildes 100 der ersten Bildfolge mit einem Merkmal 102 im reflektierten Licht bei idealer Bildgebung ohne Bildrauschen.

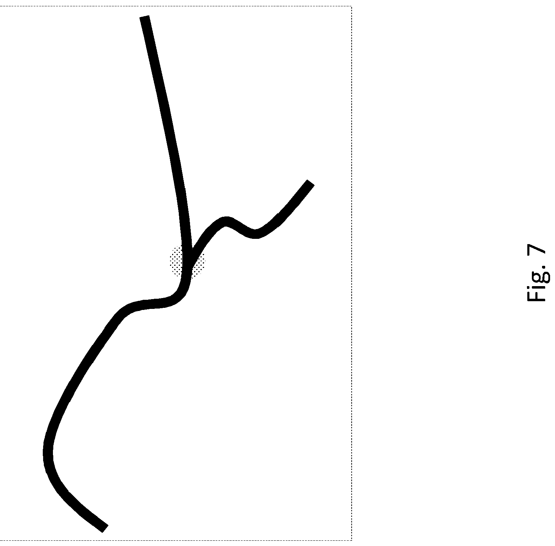

Figur 7 zeigt eine schematische Darstellung einer Überlagerung der reflektierenden Struktur 102 aus Figur 5 und fluoreszierenden Strukturen 112 bei idealer Bildgebung ohne Bildrauschen.

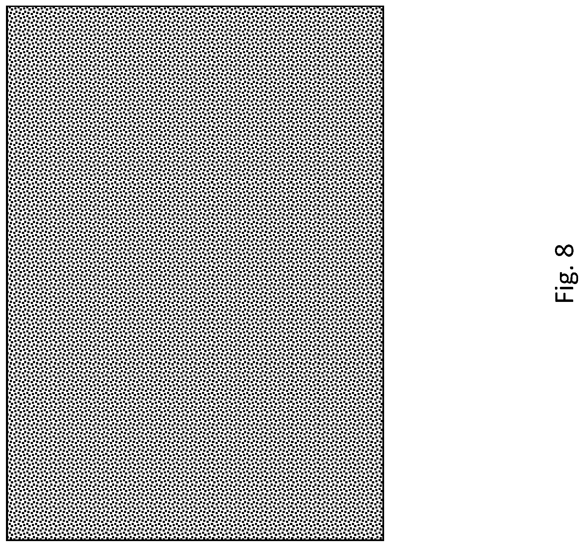

Figur 8 zeigt eine schematische Darstellung des einzelnen Fluoreszenzbildes 110 wie aus Figur 5, wie es von einem Bildsensor aufgenommen wird. Die fluoreszierende Struktur 112 ist infolge des Bildrauschens des Bildsensors nicht erkennbar.

Figur 9 zeigt eine schematische Darstellung einer Addition zweier überlagerter Fluoreszenzbilder bei idealer Bildgebung ohne Bildrauschen. Die fluoreszierende Struktur ist zweifach abgebildet, d.h. die einfache Addition zweier Fluoreszenzbilder führt zu Bildartefakten statt zu der gewünschten Verstärkung der schwachen Fluoreszenz.

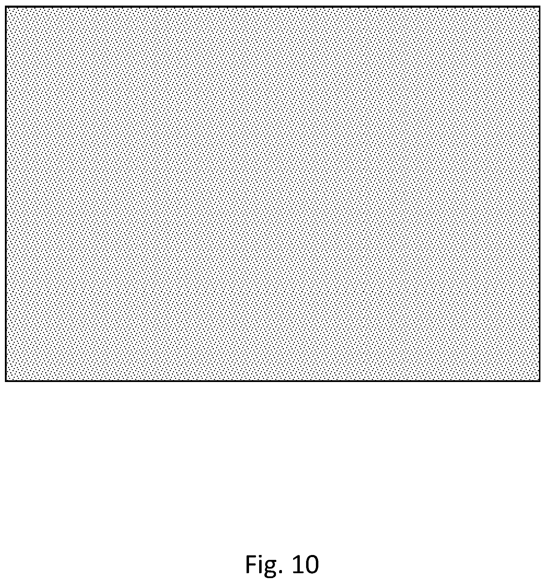

Figur 10 zeigt eine schematische Darstellung einer Addition zweier überlagerter Fluoreszenzbilder, wie sie von einem Sensor mit Bildrauschen erfasst werden. Das Rauschen des Bildes ist vermindert. Die fluoreszierende Struktur ist wegen der im Zusammenhang mit Figur 9 beschriebenen Artefakte trotzdem nicht sichtbar.

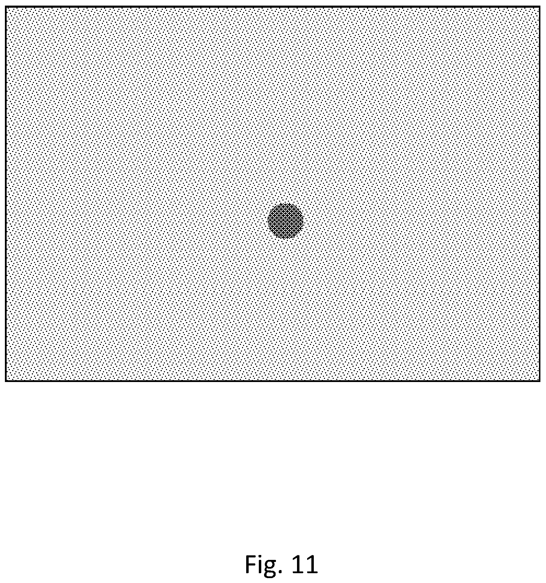

Figur 11 zeigt eine schematische Darstellung einer mit dem erfindungsgemäßen Verfahren durchgeführten, positionsrichtigen Addition zweier überlagerter Fluoreszenzbilder. Das Rauschen des Bildes ist vermindert und die fluoreszierende Struktur sichtbar.

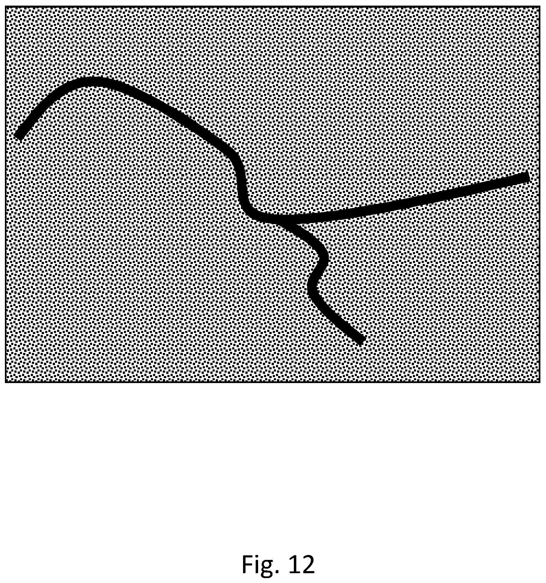

Figur 12 zeigt eine schematische Darstellung einer Überlagerung eines Einzelbildes der ersten Bildfolge, das reflektierende Strukturen zeigt, mit einem einzelnen Fluoreszenzbild, das fluoreszierende Strukturen zeigt, unter Einfluss des Bildrauschens des Sensors. Die fluoreszierende Struktur ist infolge des Bildrauschens nicht erkennbar.

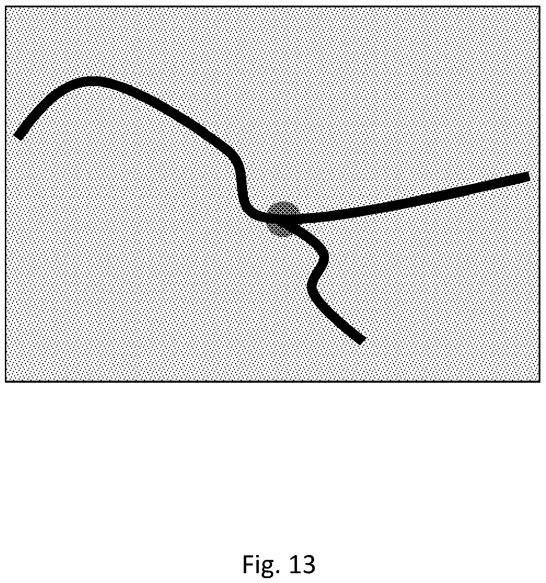

Figur 13 zeigt eine Bildüberlagerung der reflektierenden und fluoreszierenden Strukturen mit der Erfindung erzeugt. Das Bildrauschen ist vermindert und die fluoreszierende Struktur erkennbar.

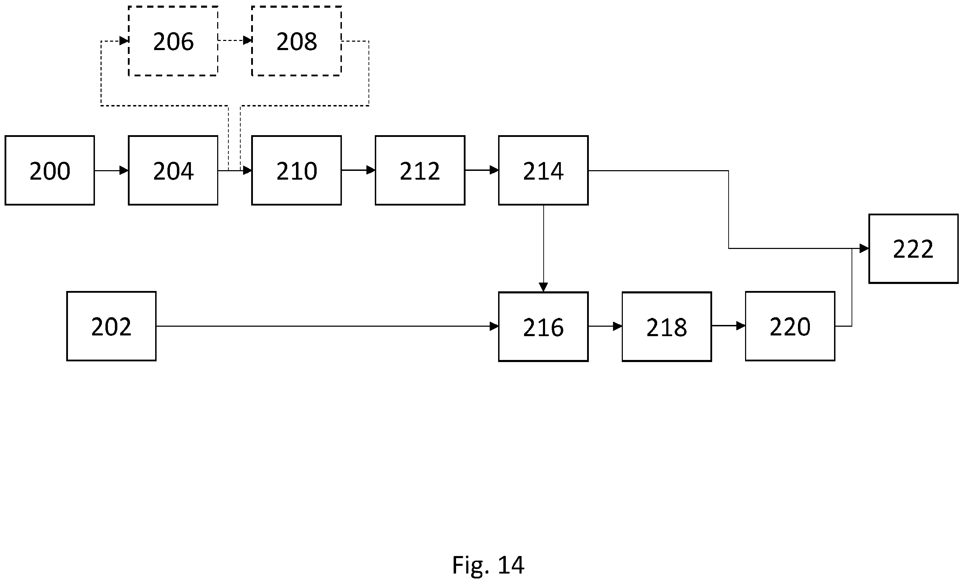

Ein erfindungsgemäßes Verfahren läuft wie folgt ab (siehe Figur 14):
Zunächst werden Bilder einer ersten Bildfolge aufgenommen (200) und gleichzeitig oder abwechselnd Bilder einer zweiten Bildfolge (202). Die Bilder der ersten Bildfolge sind Einzelbilder (Reflexionsbilder), die von dem Licht gebildet sind, das ein endoskopisch betrachtetes Objekt reflektiert. Die Bilder der zweiten Bildfolge sind Fluoreszenzbilder, die die Fluoreszenz zeigen, wenn ein Objekt mit fluoreszenz-anregender Strahlung bestrahlt wird.

Anschließend werden in den Bildern der ersten Bildfolge Objekt- oder Bildmerkmale erkannt (204) und die Merkmalspositionen der erkannten Objekt- oder Bildmerkmalen erfasst (206).

Optional kann das Erkennen von Bild- und/oder Objektmerkmalen eine Entzerrung der Einzelbilder- also eine Kompensation der optischen Verzeichnung des Endoskops - umfassen (208). Auch erfolgt vorzugsweise eine Interpolation der Merkmalspositionen (210) erkannter Objekt- und Bildmerkmale zwischen den in den Einzelbildern erfassten Merkmalspositionen, um interpolierte Merkmalspositionen für Zeitpunkte zu bestimmen, zu denen Fluoreszenzbilder aufgenommen werden.

Auf Basis der in verschiedenen Einzelbildern der ersten Bildfolge erfassten Merkmalspositionen eines jeweiligen, erkannten Objekt- oder Bildmerkmals werden Positionsveränderungen bestimmt (212). Aus den bestimmten Positionsveränderungen werden geometrischen Transformationen (Transformationsfunktionen) gebildet, die den Positionsveränderungen entsprechen (214).

Die aus den Einzelbildern der ersten Bildfolge gebildeten Transformationsfunktionen werden schließlich auf Fluoreszenzbilder der zweiten Bildfolge angewandt (216), um transformierte Fluoreszenzbilder zu erhalten in denen sich fluoreszierende Strukturen jeweils am gleichen Bildort befinden.

Die auf diese Weise transformierten Fluoreszenzbilder werden schließlich miteinander überlagert (218), um ein verbessertes Fluoreszenzbild zu erhalten.

Diese Schritte werden für jedes aktuelle Fluoreszenzbild durchgeführt, um eine Folge von verbesserten Fluoreszenzbildern zu erhalten, die schließlich dargestellt werden kann (220).

Optional kann jedes verbesserte Fluoreszenzbild mit dem dazugehörigen Einzelbild der ersten Bildfolge überlagert werden, so dass sich eine Videosequenz aus überlagerten Einzel- und Fluoreszenzbildern ergibt (222).

### Bezugszeichenliste

- 10: endoskopisches Videosystem
- 12: Endoskop
- 14: Kamerakopf
- 16: lösbare Kopplung
- 18: Objektiv
- 20: Bildsensor für sichtbares Licht
- 22: Bildsensor für Fluoreszenzbilder
- 24: Strahlteiler
- 26: Sperrfilter, sperrt Infrarotlicht
- 28: Sperrfilter, sperrt sichtbares Licht und Anregungslicht
- 30: Lichtquelle
- 32: Lichtleiter
- 34: Bildverarbeitungseinrichtung
- 36: Bildschirm

- 100, 100.1, 100.2, 100.3: Einzelbild
- 102: Objekt
- 104: Strukturverzweigung
- 106: Verschiebung
- 108: Verschiebung
- 110, 110.1, 110.2, 110.3: Fluoreszenzbild
- 112: Fluoreszenz, fluoreszierendes Objekt
- 114: verbessertes, aktuelles Fluoreszenzbild

- 200: Aufnahme einer ersten Bildfolge im reflektierten Licht
- 202: Aufnahme einer zweiten Bildfolge im Fluoreszenzlicht
- 204: Erkennung von Objekt- oder Bildmerkmalen
- 206: Bestimmung der Position von Objekt- oder Bildmerkmalen
- 208: Entzerrung der Einzelbilder
- 210: Interpolation von Merkmalspositionen
- 212: Bestimmung von Positionsveränderungen
- 214: Bildung von Transformationsfunktionen
- 216: Anwenden der Transformationsfunktionen
- 218: Überlagerung der Fluoreszenzbilder
- 220: Erzeugung einer Folge von Fluoreszenzbildern
- 222: Überlagerung von Einzel- und Fluoreszenzbildern

## Patentansprüche

1. Verfahren zum Durchführen einer Videoendoskopie mit Fluoreszenzlicht, wobei das Verfahren die folgenden Schritte umfasst:
- Aufnehmen einer ersten Bildfolge, die sich aus zeitlich aufeinanderfolgen Einzelbildern zusammensetzt, mit einem endoskopischen Videosystem, indem ein Objekt beleuchtet und das von dem Objekt reflektierte Licht als Einzelbild aufgenommen wird,
- Aufnehmen einer zweiten Bildfolge, die sich aus zeitlich aufeinanderfolgen Fluoreszenzbildern zusammensetzt, mit demselben endoskopischen Videosystem, indem das Objekt mit fluoreszenz-anregender Strahlung bestrahlt und das von dem Objekt infolge der angeregten Fluoreszenz emittierte Licht als Fluoreszenzbild aufgenommen wird,
- wobei das Aufnehmen der Einzelbilder und der Fluoreszenzbilder der ersten beziehungsweise der zweiten Bildfolge entweder gleichzeitig erfolgt, so dass jedem Einzelbild ein gleichzeitig aufgenommenes Fluoreszenzbild zugeordnet werden kann,
oder abwechselnd ein Einzelbild oder mehrere Einzelbilder und ein Fluoreszenzbild oder mehrere Fluoreszenzbilder aufgenommen werden, so dass jedem Einzelbild ein Fluoreszenzbild zugeordnet werden kann, welches mit geringem zeitlichen Versatz zum Einzelbild vor oder nach dem Einzelbild aufgenommen wurde,
- Bilden einer Transformationsfunktion zwischen verschiedenen Einzelbildern der ersten Bildfolge,
- Anwenden der Transformationsfunktion auf den Einzelbildern der ersten Bildfolge zugeordnete, aufeinanderfolgende Fluoreszenzbilder der zweiten Bildfolge zum Gewinnen transformierter Fluoreszenzbilder,
- Überlagern eines aktuellen Fluoreszenzbilds der zweiten Bildfolge mit wenigstens einem oder mehreren transformierten Fluoreszenzbildern, die aus dem aktuellen Fluoreszenzbild in der zweiten Bildfolge vorangehenden Fluoreszenzbildern gewonnen wurden, um ein verbessertes Fluoreszenzbild zu gewinnen, und
- Darstellen eines jeweiligen, derart verbesserten Fluoreszenzbilds, so dass sich eine verbesserte zweite Bildfolge ergibt, die sich aus verbesserten Fluoreszenzbildern zusammensetzt.

2. Verfahren gemäß Anspruch 1, bei dem das Bilden einer Transformationsfunktion zwischen verschiedenen Einzelbildern der ersten Bildfolge eine Erkennung von Bild- oder Objektmerkmalen und eine Erfassung der Merkmalsposition erkannter Merkmale in Einzelbildern der ersten Bildfolge umfasst.

3. Verfahren gemäß Anspruch 1 oder 2, bei dem jeden Fluoreszenzbild genau ein Einzelbild der ersten Bildfolge zugeordnet ist.

4. Verfahren gemäß Anspruch 2 oder 3, bei dem die Fluoreszenzbilder und die Einzelbilder abwechselnd und somit geringfügig zeitlich versetzt aufgenommen werden und bei dem das Bilden einer Transformationsfunktion zwischen verschiedenen Einzelbildern der ersten Bildfolge eine Inter-oder Extrapolation erfasster Merkmalspositionen umfasst, um inter- oder extrapolierte Merkmalspositionen für Zeitpunkte zu bestimmen, zu denen ein Fluoreszenzbild aufgenommen wird.

5. Verfahren nach wenigstens einem der Ansprüche 1 bis 4, bei dem das Identifizieren einer Transformationsfunktion zwischen verschiedenen Einzelbildern der ersten Bildfolge eine Korrektur der optischen Verzeichnung des endoskopischen Videosystems (Entzerrung) umfasst.

6. Verfahren nach wenigstens einem der Ansprüche 2 bis 5, bei dem das Erkennen der Bild- und/oder Objektmerkmale in den Einzelbildern der ersten Bildfolge dadurch vereinfacht wird, dass eine Positionsveränderung von Bild- und/oder Objektmerkmalen zum letzten Einzelbild der ersten Bildfolge indirekt aus den Positionsveränderungen erkannter Bild- und/oder Objektmerkmale zwischen zeitlich direkt aufeinanderfolgenden Einzelbildern der ersten Bildfolge ermittelt wird.

7. Verfahren nach wenigstens einem der Ansprüche 1 bis 6, bei dem das endoskopische Videosystem ein Stereoendoskop umfasst, so dass sich ein Einzelbild der ersten Bildfolge und ein Fluoreszenzbild der zweiten Bildfolge jeweils aus einem linken und einem rechten Halbbild zusammensetzt, wobei die Merkmalspositionen in beiden stereoskopischen Halbbildern mit dem Verfahren gemäß der Ansprüche 1 bis 6 verbessert werden.

8. Verfahren gemäß Anspruch 7, bei dem in zwei zeitgleich oder zeitnah aufgenommenen stereoskopischen Halbbildern der ersten Bildfolge Merkmalspositionen erfasst werden, die zu demselben Objektmerkmal gehören und daraus eine stereoskopische geometrische Transformation vom linken zum rechten stereoskopischen Einzelbild berechnet wird.

9. Verfahren gemäß Anspruch 7 oder 8, bei dem eine Addition oder Mittelwertbildung zwischen dem linken und dem rechten Fluoreszenzhalbbild der zweiten Bildfolge durchgeführt wird.

10. Videoendoskopiesystem mit Bildverarbeitungseinrichtung, bei welchem das Videoendoskopiesystem dazu konfiguriert ist, zwei Bildfolgen aufzunehmen, und zwar eine erste Bildfolge, die sich aus zeitlich aufeinanderfolgen Einzelbildern zusammensetzt, wobei ein Objekt beleuchtet und das von dem Objekt reflektierte Licht als Einzelbild aufgenommen wird, und eine zweite Bildfolge, die sich aus zeitlich aufeinanderfolgen Fluoreszenzbildern zusammensetzt, wobei das Objekt mit fluoreszenz-anregender Strahlung bestrahlt und das von dem Objekt ausgehende Licht als Fluoreszenzbild aufgenommen wird,
und die Bildverarbeitungseinrichtung dazu konfiguriert ist,
- ein oder mehrere Einzelbilder der ersten Bildfolge einem oder mehreren, wenigstens annähernd gleichzeitig aufgenommen Fluoreszenzbildern der zweiten Bildfolge zuzuordnen,
- aus den Einzelbildern der ersten Bildfolge Transformationsfunktionen zu bilden, die die geometrische Veränderung von Bildmerkmale zwischen zwei Einzelbildern beschreiben,
- die abgeleiteten Transformationsfunktionen auf die den Einzelbildern entsprechenden Fluoreszenzbilder anzuwenden und
- die auf die Weise transformierten Fluoreszenzbilder zu einem verbesserten Fluoreszenzbild zu überlagern.

11. Videoendoskopiesystem gemäß Anspruch 10, bei dem die Bildverarbeitungseinrichtung eine Grafikprozessoreinheit (GPU), eine anwendungsspezifische integrierte Schaltung (ASIC) oder ein feldprogrammierbares Gatearray (FPGA) aufweist.

12. Videoendoskopiesystem gemäß Anspruch 10, welches ein Stereoendoskop aufweist.

13. Videoendoskopiesystem gemäß Anspruch 10 oder 11, welches ausgebildet ist, ein Verfahren gemäß Anspruch 1, 2, 3, 4, 5 oder 6 auszuführen.

14. Videoendoskopiesystem gemäß Anspruch 12, welches ausgebildet ist, ein Verfahren gemäß Anspruch 7, 8 oder 9 auszuführen.

15. Verwendung eines Videoendoskopiesystems gemäß einem der Ansprüche 10 bis 12, zum Durchführen eines Verfahrens gemäß einem der Ansprüche 1 bis 9.
